# EUROPEAN PATENT SPECIFICATION

(11) **EP 2 054 840 B1**
(45) Date of publication and mention of the grant of the patent: **03.10.2012**
(21) Application number: 07786651.5
(22) Date of filing: 10.08.2007
(51) Int. Cl.: G06K 9/00, G07C 9/00

(54) **METHOD TO TRANSMIT PHYSIOLOGICAL AND BIOMETRIC DATA OF A LIVING BEING**
VERFAHREN ZUM SENDEN PHYSIOLOGISCHER UND BIOMETRISCHER DATEN EINES LEBENDEN WESENS
PROCÉDÉ DE TRANSMISSION DE DONNÉES PHYSIOLOGIQUES ET BIOMÉTRIQUES D'UN ÊTRE VIVANT

(30) Priority: 16.08.2006 DE 102006038438; 04.04.2007 US 732225
(43) Date of publication of application: 06.05.2009
(73) Proprietor: Keppler, Bernhard, Westport, CT 06880 (US)
(72) Inventor: Keppler, Bernhard, Westport, CT 06880 (US)
(74) Representative: Blickle, K. Werner
(86) International application number: PCT/EP2007/007080
(87) International publication number: WO 2008/019800

(56) References cited:
- WO-A-03/075227
- WO-A-2004/100101
- US-A- 4 999 613
- US-A- 5 889 474
- US-A- 6 104 913

## Description

### FIELD

The invention relates to a method for secure transmission of information regarding. The identity of an individual and physiological characteristics of that individual.

### BACKGROUND

Biometrics or biometric science is the science of using biological properties to identify individuals; for example via fingerprints, retina scans, voice recognition, facial recognition and the like. For human individuals, their fingerprint is most often used for biometric identification. Many applications are known for fingerprint identification. Reference is made to U.S. Pat. No. 6,484,260 issued to Identix for Capacitative Fingerprint Recognition; U.S. Pat. No. 6,404,904 issued to TST for Touchless, Optical Fingerprint Sensor; U.S. Pat. No. 6,483,932 issued to Crossmatch for rolled Fingerpint Identification; International Biometric Group Industry Report 2006-2010, Biometric Consortium "Introduction of Biometrics", John Wiley & Sons publication "Biometrics: Identity Verification in a Networked World" by Smair Nanavati, Michael Thieme and Raj Nanavati.

For certain applications, the apparatus can be miniaturized to the extent that an individual can carry it as a portable device. Such a device can transmit the recorded biometric data identifying the user via fingerprint identification to a receiver and thereby allow the validation of the user's access control level to use or operate e.g. a computer, enter a locked vehicle or a secure facility. Such a device is described for example in U.S. Pat. No. Patent 6,850,147. The range of functionality for those devices is very limited, requiring separate devices specifically designed for different applications. Transmission of the data can be accomplished via Near Field Communication (NFC) using technologies such as RFID, Bluetooth Technology or others. U.S. Pat. No. Patent 6,754,472 describes an alternative technology for the transmission of those data using the electrical conductivity of the human skin.

Apparatus is known which can record physiological data and/or conditions of a living being including units for invasive and non-invasive examination of a living being. They include by way of example, blood tests, DNA definition, blood pressure measurement, heart beat frequency and many others. Today, many apparatus for non-invasive examination are available in portable form. For example, reference is made to apparatus for measuring EKG or apparatus that can be wrist-worn measuring blood pressure and heart beat frequency, for example, during physical exercise.

Finally, ID cards with integrated memory chip issued by health insurance agencies can assist with patient identification during doctor's and/or hospital visits. Some cards allow storage of certain historic physiological information of the user. However those ID cards are very limited in their storage capacity and are not sufficiently tamper proof to meet federal privacy requirements.

In German Utility Model application DE 20 2005 020 535 U1, Kowatsch describes a device including a biometric sensor and a sensor for physiological information, for example heart rate. However, this device is less secure than desired. It must be manually activated and deactivated.

In U.S. Pat. No. 5,876,926 there is described a method including the steps of collecting a sample from a test subject and taking biometric data from said test subject. The sample can be labeled with information including the biometric data.

WO 03/075227 A1 describes a method and a device for detecting the presence of an individual in a predertermined place and verifying the identity of said individual to be guarded for reasons of public security interest, such individual, in particular, being a parolee. The known device taking the form of a bracelet secured to the wrist or ankle of the parolee receives a verification signal sent by a central computer unit under control of a guard of a prison or security service. The parolee is then requested to submit biometric data, such as a predetermined fingerprint, an image of the retina or a voice pattern of his, as well as transmit data of presence, such as his heart beat or body temperature, by way of respective sensors comprised by said device. In receipt of the confirmation signal, said guard checks whether the bracelet is in a predetermined area, such as the home of the parolee to which he is confined by order of a court. First and foremost, this known method and device are concerned with a more or less continuous, GPS-controlled observation of the "confined" position of the device. The request for verifying the identity of the parolee, obviously, is much more sporadic in nature. This can lead to misuse in-between consecutive requests for data of identity. The data of presence in form of heart beat or body temperature appear to be merely used as digital information, i.e. in the form of "present" / "not present".

WO 2004/100101 A1 discloses a method very similar in nature to the aforementioned WO 03/075227 disclosure. The device used differs in so far as it consists of two units, namely a mobile control unit and a bracelet for the parolee. To control tampering, data of presence are transmitted from the bracelet to the mobile control unit at predetermined intervals. If the step of verification of the presence of the parolee fails, an alarm signal emitted by the mobile unit is received by a central monitoring station for further action. This known method, again, is mostly concerned with calculating precise position data of the mobile unit. Data of presence are strictly used to determine: is there a pulse "yes" or "no" and/or is there a temperature "yes" or "no"?

US 5889474 A discloses an earlier method very similar to the disclosure of the two preceding references. It is the central object of this patent to provide efficient, accurate, robust and low cost means and methodology for providing two way data packet messaging that support continuous custodial electronic monitoring (CCEM) services that will operate within all cellular mobile radio systems, and personal communication systems control and access channels. The disclosure concentrates on a method and device to be used for monitoring one or more individual under home arrest using existing digital access and cellular digital control channels as a means and methodology to transmit and manage data packets that contain information that reveals home arrest subject behavioral status, identification, and current location information. The reason for providing a communicator device that takes the form of a foldable cellular phone including a hidden GPS antenna is to ease assimilation of criminal offenders, who seek successful integration into society without having to utilize a seperate and distinct looking home arrest communicator. The arrangement requires that the offender wear a leg band tightly secured to a limb, with a radio transmitter of said leg band staying in radio communication with the communicator device. For gathering further information about the offender, such as illegal drugs and unauthorized alcohol content in the blood of the offender, blood hormones, etc., the known method requires a biometric implant within the body of the offender in close proximity to the leg band. Such an implant in a human being is hardly acceptable under conditions other than those based on a court order. While the disclosure makes reference to conceivable variations of said method, reduction to practice for any of these is neither suggested, nor described in detail.

US 4,999,613 A describes a remote confinement system similar to, but not as far as sophisticated as those referred to above. A plurality of tests are provided to determine the presence, namely by a continuous RF test, the identity, by a speach test, and the conduct of a prisoner by an alcohol test. The patent is mostly concerned with providing a configuration that reduces the number of service calls, conserves the amount of online time, reduces the time of central unit personnel to a minimum and increases the number of remote units which may be serviced by a particular central processing center of a home arrest system. Online time, e.g., is reduced by reducing the number of reports of departure by the prisoner when in fact the departure signals are due to inadvertent or temporary loss of signal from the transmitter, and not from true departures of the prisoner from the remote location. It may take as many as five minutes before a prisoner is assumed to be missing.

US 2003 / 0174049 A1 describes a method and an identification appliance, such as a wrist band, for providing information about an authorized bearer including name, address and phone number as well as biometric data, e.g. fingerprint data, medical data and the like. Starting from the fact that it is known to electronically collect patient information, assure facility access control or track the location of personnel, such as convicts in a prison, the document is first and foremost concerned with more effective communication between the appliance, in particular its data storage device, and a wireless communication network. It is being suggested e.g. to share the entire medical history of an authorized bearer with other people, who are free to add to, delete from or modify the information or monitor the location and condition of a patient within a hospital, respectively an outpatient setting to enable healthcare professionals and family members to monitor a patient's health status and to remain in contact via data, alarms, or by two-way voice communication. In light of the fact that the disclosure is concerned with the most effective and unrestricted way of sharing information of an authorized bearer, little, if anything is mentioned about how to guard information against manipulation by third parties and misuse. In order to make the appliance "secure" it is suggested that it be configured to make removal or tampering difficult or impossible or, alternatively, provide a tamper-evident function, that is indicate whether tampering of the appliance was attempted. If the latter is true, a display or alarm is activated or a person or another device is informed.

The present invention is concerned with providing a solution to a data security problem present in the prior art through a method that can be enabled by a multi-functional and interoperable apparatus to be used to improve and provide a higher security level for access control and operator authorization and to allow an external data transmission of the recorded biometric and physiological data tailored to its user and inseperably connected to the identified user of the apparatus without the possibility of interfering with or manipulating the data.

### SUMMARY

Starting from the disclosure in WO 03/075227 A1 the aforementioned data security problem is solved for a method in accordance with the preamble of claim 1 by determining that an external eceiver has received said signal, by terminating transmitting if said signal has not been received for a predetermined amount of time and by resuming transmission on the condition that breach of a security condition has been rectified and that the individual reauthenticates with biometric information.

### BRIEF DESCRIPTION OF THE DRAWINGS

The invention submitted is described in more detail and its functionality explained by a reference to an embodiment thereof. An apparatus useful for carrying out the invention is shown in the accompanying drawings.

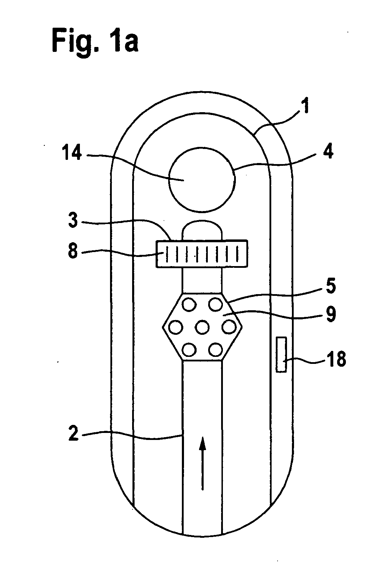

Fig. 1a is a schematic overview of a multi-functional apparatus;

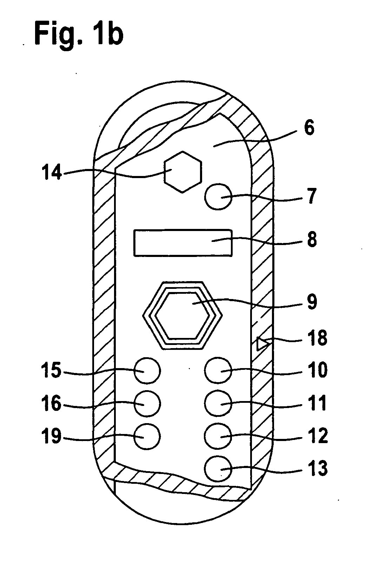

Fig. 1b is a cut away view of the apparatus as shown in Fig. 1a.

### DETAILED DESCRIPTION

A particularly advantageous embodiment of the apparatus to carry out the method of the invention includes 1) an off-the-shelf fingerprint sensor (See Nanavati et al cited above) which can be used to determine access rights and authorization levels and 2) an optical physiological data sensor for multi-spectral analysis of the user's subcutaneous physiological conditions 3) an apparatus for sensing a parameter of a security condition and 4) a communication chip. The optical physiological data sensor is known and allows the measurement of physiological data from subcutaneous tissues of an individual. Sensing blood alcohol content is particularly important. The preferred apparatus for sensing a security condition is one that measures the distance between the sensor and the user's skin. The transmission of the recorded biometric and/or physiological data to an external receiver can be accomplished with a known communication chip integrated into the apparatus and using RFID. The method is particularly useful in controlling access to chemical manufacturing machines, particularly machines that make pharmaceutical compositions.

Available technology today offers very high degrees of integration of components and therefore can allow for the method to be performed by an integrated apparatus to be worn by the user. The apparatus can be a multifunctional apparatus similar to watches, cell phones, cameras with the purpose of identifying and authenticating the user for the purpose of determining access rights and authorization levels to a facility or machine and/or monitoring bodily functions of the user in real time. The latter data can be displayed on a display integrated with the apparatus in whose memory historic data can be stored which for example can be transmitted to the external receiver during a doctor's visit together with the identity of the user. The apparatus for the last mentioned application can replace medical ID cards and can facilitate and improve the flow of physiological data between patient, doctor, insurance company and pharmaceutical industry. It can also be completely protected against misuse and fraud. The method of the invention can also be accomplished using a stand-alone or portable apparatus to determine access authorization to a physical facility or machine. Insurance companies will be interested to assure that only authorized and persons meeting current training requirements will be able to operate machinery posing serious consequences in case of accidents. The apparatus for accomplishing the method of the invention can limit the use of such machinery, if required, to only one person. Such a person might also be required to demonstrate in real time that he/she currently has the physical condition to operate such machinery. The invention can provide such verification real time through its optical sensor and ability to record, compare and store physiological information such as heart rate, blood alcohol level etc. The apparatus used to accomplish the method might take a stand-alone form for those applications while a wearable apparatus can be used when the user's mobility is essential. If, for example, a policeman or security guard or a person with similar responsibilities is on patrol and has the wearable apparatus on his/her body, the method can transmit a distress code to a central station as soon as the apparatus detects a medical condition such as very high heart rate, indication that the user is exposed to a stress or threat condition.

The method according to the invention further provides a multi-functional system to store physiological data of an individual. Physiological data can be recorded continuously on storage media in the apparatus. Such inventive system can combine separated functions or apparatus into a single apparatus, inseparably linked to a single person and which can be used in a large number of applications. In another aspect, the method of the invention can include an automatic, inseparable connection between biometric and physiological data and the living being, particularly its user. The inseparable connection between the biometric data and the physiological data can be accomplished with software embedded in a processor or in the communication chip. The software can place biometric data and physiological data in a predetermined file format. This file format can further accommodate encryption. To accomplish the same with today's apparatus requires additional staff and/or multiple apparatus for the recording, storage and processing of the data. The method according to the invention can therefore be more economical than any apparatus or method used to date.

To achieve the highest possible guarding of the data recorded, a communication chip can be integrated with the apparatus and can automatically terminate all external data communication if certain security conditions are not met. For example, if the apparatus is placed at a distance to its user's skin greater than a value which can be user or factory set, external data communication can be terminated. This prevents any manipulation by third parties and misuse of the invention. If transmission has been terminated as described above, only its biometrically identified user(s) or a previously defined authorized individual or if configured in this way, during emergencies, a pre-determined person can re-activate the invention via biometric re-validation and authorization, for example re-scanning the fingerprint data. Other examples of security conditions that can be used to terminate communication include a change in temperature of the area surrounding the apparatus; a lack of motion of the apparatus for a predetermined amount of time; movement of the device outside a predetermined area as measured by an on-board GPS receiver, failure to receive a communication from an external transmitter and other conditions. For these embodiments, the apparatus to accomplish the method of the invention can contain other sensors such as temperature, motion sensors, GPS receivers and other receivers and the like. The apparatus can also be configured to receive signals from external sensors to terminate communication.

The method can also include the step of receiving information from an external transmitter in addition to the information from the external sensors just mentioned. General information such as name of user and also sets of comparison values used when recording biometric and/or physiological data can be received from an external transmitter or transmitters. In addition to the physiological data and the biometric data that is sensed according to the invention, other data can be stored on the device and can also be transmitted. Examples of other data that can be transmitted include medical history data, including dental records and the like. Another example of data that can be transmitted is information regarding the training history of the individual.

In the context of controlling access to a chemical manufacturing machine, the method can provide for the transmission of identity information through biometric data, blood alcohol content, information that the individual has received all of the current safety training necessary and has been trained in the operation of the particular machine. The machine receives this transmission and if acceptable, commences or continues operation. The machine can also be programmed to transmit back to the device that the individual is wearing indicating status.

The apparatus used to accomplish the method of the invention can be capable of usage by multiple living beings, each one having to perform the above referenced biometric and physiological enrollment to be authenticated for its use.

The apparatus according to the invention can further provide a method for the continuous biometric and/or physiological identification of the living being. Specifically, the invention's method can compare currently recorded physiological data with historic physiological data of the user and trigger visual or acoustical signals if the difference of currently recorded physiological values compared to historic values lies outside pre-defined ranges. If this method is applied primarily to human beings, the invention can be configured such that the information for at least one medical condition are stored and externally transmitted only during pre-determined conditions (i.e. during a doctor's visit, after an accident, if the person has passed out etc.). In such cases the external receiver (stationary at the doctor's office or mobile with the EMS crew) can send a special code set to the integrated communication chip that the invention's method can accept and trigger a release of physiological and other data from its memory.

In accordance with the method of the invention, sensing the biometric characteristics of an individual is one step. A wide variety of biometric sensors are known and can be used to accomplish this step.

Biometrics allow for the definition of characteristic identifiers of living beings including animals. The following description of the invention uses from the large amount of biometric identifiers available as an example the fingerprint of human beings. The identification of fingerprints is a well-known technology and is embodied in a law enforcement system referred to frequently as AFIS (Automated Fingerprint Identification System). Fingerprints can be recorded with a variety of sensors (capacitive, optical, thermal, acoustical, pressure sensitive and the like). The invention is however not limited in its application to human beings or fingerprint identification. Any other biometric data can be used including, without limitation, cutaneous pattern data, facial feature pattern data, blood vessel pattern data, iris pattern data, data from a person's sweat, voice pattern data and the like.

Reference is made in particular to the Lumidigm Inc., Albuquerque, New Mexico, USA. Their optical physiological data sensor uses the multi-spectral range and is used to record data related to the skin of a person. The Lumidigm sensor (Lumiguard®) provides skin data on the surface as well as subcutaneous. In particular, wavelengths in the near infrared spectrum are being used, which are being reflected by the subcutaneous tissue of the person. The special feature of this optical sensor is that it not only records the fingerprint of the person with a much higher accuracy compared to other fingerprint sensor technologies but this optical sensor can also record physiological data of the person. In particular the recording of blood sugar levels and hemoglobin values can be accomplished. This allows deploying the sensor for a multitude of applications. Sensormag/Motionnet are manufacturers/distributors of a variety of sensors that can be integrated into the apparatus used in the invention for recording a variety of physiological data. EP1217948B1 of Lumidigm, Inc. describes a method for the determination of a person's identity via comparison of the same person's stored spectral tissue data records with the current spectral tissue data.

Another step of the method of the invention is comparing the characteristic data of the individual with predetermined registered individuals and determining whether the individual is registered. The predetermined registered individuals register themselves in a setup step or a database of registered individuals can be stored on a device for later comparison. Comparison of the sensed biometric data of an individual with those registered can be accomplished with a simple computer program.

Another step of the method of the invention is sensing a physiological characteristic of the individual. A wide variety of physiological sensors are known in the art and can be used to accomplish this step. It is possible to use the sensors on a sample taken from the individual but it is preferred to use non-invasive technology so that no sample is necessary.

For example, a large body of literature deals with the non-invasive measurement of glucose levels in humans. An apparatus for glucose monitoring is known using standard power supply and including a glucose detector element and thermal gradient spectrophotometer. Methods are known for the non-invasive measurement of the presence of and density of blood components. Subject to measurement are hemoglobin, cholesterol, albumin, alcohol and glucose. U.S. Pat. No. 6,067,463 also describes such an apparatus and method using two measurements with electro-magnetic frequency of similar but not identical wavelength. Analyzed substances include glucose, urea, creatine, ketone, bilirubin, hemoglobin, urobilinogin and protein. Additional publications can be found under Chemical Chemistry 2003;49: 924-934. "Monitoring Blood Glucose Changes in Cutaneous Tissue by Temperature-modulated Localized Reflectance Measurements" and under OPTICS LETTERS / Vol. 19, No. 24 / December 15, 1994 titled "Influence of Glucose Concentration on Light Scattering in Tissue-Simulating Phantoms".

In the article: Diabetes Technology & Therapeutics Noninvasive Blood Glucose Measurements by Near-Infrared Transmission Spectroscopy Across Human Tongues May 2000, Vol. 2, No. 1 : 5 -16 Jason J. Burmeister, Mark A. Arnold, Gary W. Small, there is described a Europe-wide certified apparatus from Pendragon Medical manufactured in the form of a wrist watch, performing non-invasive glucose measurements continuously on the basis of impedance spectroscopy. Changes in the glucose levels of the blood triggers changes of the electrical characteristics of the skin und the subcutaneous tissue. Changes between the electric fields of the skin and its underlying tissue are being measured by the "sugar watch" clock and recorded by a sensor on the bottom of the apparatus. Changes are being recorded and stored up to one month in the apparatus.

In another step of the method of the invention, the physiological data is compared with a predetermined acceptable range for that data. For example, where the invention is used to prevent the operation of machinery when the individual is intoxicated, the physiological data is blood alcohol data and the predetermined range is the upper limit beyond which the individual will not be allowed to operate the machinery. This is accomplished by installing a receiver at the machinery that will not allow said machinery to start unless it receives a secure code from the communication chip according to the method of the invention. Such secure code may follow the determination of the type of machinery to be operated and a sequential check of the users credentials including biometric identification, predetermined physiological conditions to be within predetermined ranges and additional authorization elements such as completed training to operate the machinery etc.

In another step of the method of the invention a parameter characteristic of a security condition is sensed. Breach of security (detecting a security parameter outside a predetermined range) can be detected by sensing the distance between a sensor in the device and a wearer's skin. A receiver and transmitter and an oscillator can be provided to continuously sense the strength of the capacitive field surrounding the body of the user of the apparatus. A controller chip can continuously receive the values of the capacitive field and forward them to the processor of the apparatus. The apparatus' processor compares the values received with the preset value and can generate commands to the communication chip to stop all external data communications if the preset value is exceeded. Thus, data will not be transmitted unless the individual is registered, as confirmed by biometric data; the physiological data falls within the allowed range and the security condition is satisfied.

Breach of security can be a detected by a change in temperature. For example, a device that is intended to be worn can include a temperature sensor that measures the temperature of the wearer's skin. If the device is removed, the sensor will detect a lower temperature than programmed and will terminate communication. The temperature sensor can also be used to detect ambient temperature and the device can be programmed to terminate communication of the device is removed to a new environment. For example, a device intended for indoor use can terminate transmission if the temperature falls outside the normal indoor temperature range.

Breach of security can be detected by a change in motion characteristics. For example, again with a device intended to be worn, detecting absence of motion over a predetermined period of time can be indicative of the removal of a device from a wearer or that the wearer may have fallen asleep or otherwise be unconscious. Such a condition would result in the termination of communication.

Breach of security can be detected by a change of location. For example, a device can include a GPS receiver and the position data from the receiver can be used to compare the position of the receiver to allowed position information stored in the device. If the device is not in an approved area, the communication can be terminated.

In all situations where security has been breached, the device can be programmed to resume communication a) when the security condition has been rectified and b) when the user reauthenticates with biometric information.

In another step of the method of the invention, a transmission is made based on the results of the sensing of the biometric data, the sensing of the physiological data and the determination of a security condition. A wide variety of wired or wireless transmission systems can be used. For example, RFID (Radio Frequency Identification) systems can be used. Active-passive and active-active Near Field Communication (NFC) protocols based on RFID are short range wireless communication technologies that are useful in the invention. This communication typically operates in the 13.56 MHz band. This technology is extremely well known and documented and is embodied in numerous standards and is extensively described in the literature and in patent specification. Similarly, Bluetooth® technology is a short-range communication system that is useful in the invention and operates in the 2.4 GHz band. It also is extremely well known and documented.

Wider range communication technologies can also be used such as WiFi and WAN technologies. Wired communication technologies such as Ethernet and USB are also useful. Numerous manufacturers are developing new communication chips for data transmission over the human skin in many parts of the world. In Japan, NTT is developing a communication chip according to an article in the VDI Nachrichten.com dated 05/12/2006 under the name "Red Tacton". A communication chip called "Skinplex" is already being marketed by Ident Technology AG, 82234 Wessling, Federal Republic of Germany. Applications for "Skinplex" include security when accessing an automobile, automatic settings when entering an automobile and others. Deployment of "Skinplex" to prevent objects such as a human hand from getting caught by a closing car sunroof has already been completed by the company Inalfa Roof Systems, Holland in 2005 according to the magazine "Automobilwoche" No. 26 of 09/26/2005. The complementary receiver can consist of any conducting material that responds to the capacitive field generated by the human skin. If a human carrying the communication chip near his skin or a limb enters this capacitive field, a small capacitive change is recorded and the data transmission between communication chip and receiver is activated. Other examples of disclosures relating to the transmission of data over the human body are U.S. Pat. Nos. 5,796,827; 6,104,913; and 6,211,799.

In Fig. 1, there is shown a schematic overview of a multi-functional apparatus that can be used to carry out the method of the invention to record, store, compare and method physiological and/or biometric data of a living being. The useful apparatus is described in figures 1a to 1b. The apparatus consists of an enclosure 1 made of firm material, and integrates a finger guide 2 to guide the user's finger over the fingerprint sensor 8 (the sensor for biometric data in this embodiment). The enclosure 1 includes enclosure opening 3 for a fingerprint sensor apparatus 8. The enclosure can also include a functional display 14 and an enclosure opening 5 for a physiological data sensor 9, in this embodiment an optical sensor. The enclosure 1 can also include an opening (not shown) for the security sensor 19. Also shown is switch 16 used to turn the device on or off and transmission switch 18 which can be used to manually start/stop data transmission. A data port (not shown) can also be included.

As shown in Fig 1b, inside the enclosure 1 is a circuit board 6 and power supply 7. The power supply 7 can be a battery, in particular a high-energy battery as used in consumer electronics. As an alternative, a solar chip (not shown) may be used to charge the battery or recharging can be supplied via USB connection, for example through data port (not shown). Powering the apparatus may be accomplished also via energy generated through movement as is being offered on many wristwatches (not shown).

The circuit board 6 consists of several subassemblies including fingerprint and physiological data sensors 8 and 9, a processor 10, an algorithm 11 to interact with the processor 10, a memory module 12, a matcher software module 13 and a communication chip 15. Also shown is security sensor 20.

The fingerprint sensor module 8 can include the software developed and licensed by Ikendi Software AG, 82205 Gilching, Federal Republik of Germany or other sensor modules from many manufacturers of fingerprint sensors. The first sensor module can be the swipe fingerprint sensor manufactured by Atmel Inc., San Jose, CA, United States of America. Reference is made to Atmel White Paper: "Atmel's FingerChip™ Technology for Biometric Security" 3312A-BIOMT-11/02. Other comparably miniaturized sensor modules to measure, method and compare other unique biometric characteristics of the user, i.e. facial recognition, iris pattern; retina detection, blood vessel recognition recorded via camera module as being used in cell phones can also be also used in the apparatus.

The apparatus can be integrated into a stationary apparatus or in general become an element of a apparatus for daily use, such as a mobile phone, particularly those containing a camera module, or a digital camera, a wrist watch, PDA, PC, credit card sized device or other apparatus. In the case of a camera phone or digital camera the fingerprint sensor 8 can be connected with the camera module (not shown) and the physiological data sensor 9 can be connected with its memory module 12 to store historic physiological data of the user.

The physiological data sensor module 9 can consist of an optical sensor operating in the multi-spectral range retrieving information about the spectral data of the user's tissue. The preferred optical sensor is the one manufactured by Lumidigm Inc., Albuquerque, NM, United States of America. This sensor can record information about hemoglobin und glucose levels of the user in non-invasive mode. In addition, know-how derived from the apparatus as described in U.S. Pat. No. 6,067,463 that can additionally record levels of urea, bilirubin and protein can be utilized within the apparatus. Alternatively, the physiological data sensor 9 can be an ultrasonic controller that records the sound waves reflected by the user's subcutaneous tissue and can be interpreted and stored as physiological data of the user.

The apparatus can integrate a variety of sensor components which record physiological data of the user via optical or acoustical signals sent to the subcutaneous layer of the user and interpret the reflected signals as the status of a predetermined physiological condition, i.e. blood sugar, blood alcohol et al. The physiological data sensor 9 can record such data on a continuous basis, or by request from the user by pressing a function key (not shown) on the apparatus or via command received from an external reader/transmitter operated for example by a doctor, emergency physician etc.

The value of any such physiological data element can be calculated within the sensor 9 and forwarded continuously to the apparatus' processor 10. The processor 10 can compare the received values with pre-determined values, for example stored in memory 12, and generate a visual or audible message to the user if such received value exceeds a pre-set range, i.e. level of blood sugar, blood alcohol level higher than legal limit.

Example for the function of the matcher 13 from Ikendi GmbH in Gilching, Federal Republic of Germany, for the fingerprint sensor 8 manufactured by Atmel Inc.

The display 14 can consist of a simple LED display or liquid crystal panel to show the results of the authentication of the user based on the result of the evaluation of the biometric and/or physiological data of the user. This information can be displayed simply as "Y" or "N" or in conjunction with a set of pre-stored messages in the memory of the apparatus. Based on the primary application of the apparatus, the display 14 may prompt the user to re-identify himself or re-record physiological data. If the apparatus is integrated into a mobile phone, requests for password or similar entries can be pre-programmed. Displaying physiological data can be more user-friendly if a liquid crystal is being used.

In one embodiment, the LED signals from LED 14 can be as follows:
- LED light blinking green: medical data are being transmitted and/or received by the apparatus;
- LED light solid green: fingerprint enrolment or fingerprint identification has been accepted;
- LED light solid red: fingerprint enrolment or fingerprint identification has been rejected due to incomplete data or invalid user condition
- LED light blinking orange: request to user to enroll a new fingerprint template. For enrolment, the user has to place/swipe the finger three times to generate a valid biometric template.

If a display is being used instead or in addition to the LED, the messages displayed on it will be compatible with the above descriptions for the various LED conditions.

The communication chip 15 can be a commercially available apparatus manufactured by several companies internationally. Communication chips using Near Field Communication are particularly suitable for the apparatus including RFID (radio frequency identification), Bluetooth® technology or communication chips using the above described data transmission over the skin. This latter technology uses the electro-magnetic field surrounding the skin of each person as data channel for near field communication. The communication chip for this technology receives and transmits data over the human skin using weak electrical fields completely harmless to the individual. Data transmission using this technology is tamper proof as compared to the first two mentioned methods for near field communication. The data stream between communication chip and external receiver can be encrypted to further enhance security and privacy of the user. Although using very low signal strength the data transmission is superior, less costly and less power consuming than the alternative radio frequency technology. External disruptive conditions such as water, changes of temperature etc. do not interfere with this data transmission technology.

A particular advantage of the apparatus is derived if the communication chip 15 is linked with a switch-off trigger 16 that automatically deactivates the apparatus if there is a security breach, as previously discussed, based on a signal from security sensor 19. Automatic deactivation can occur after elapse of a pre-determined amount of time or if the user places the apparatus at a greater distance from his/her skin than configured for the particular apparatus. If the apparatus is being accidentally misplaced, lost or stolen, any misuse of the apparatus is prevented. To re-activate the apparatus, the user must re-identify himself/herself via the fingerprint sensor 8. For special applications, several individuals can be enrolled with their fingerprints as authorized users.

In the embodiment shown in Fig. 1, the security sensor consists of a signal generator that is worn close to the human body and monitors changes in the capacitive field surrounding the human skin. It calibrates itself for each user of the apparatus and if it senses a change of the capacitive field outside a pre-set range, it will shut off the apparatus and will only function again after the holder has identified himself/herself biometrically.

An alternative security sensor can utilize a similar process to sense changes in temperature surrounding the apparatus or sensing the elapsed time of inertia of the device. If the temperature sensor records a new value outside a pre-set range and/or if the inertia sensor records elapsed time greater than a pre-set value, the security sensor sends a signal to processor to shut down the apparatus.

The apparatus can be also programmed in a way that the first person or the first number of predetermined initial persons who are enrolling themselves biometrically are considered master users. Under certain security conditions, after the apparatus has terminated the external data transmission, the apparatus can only be re-started if one of the master users is first validated biometrically on the apparatus followed by the biometric validation of the current user.

In one embodiment, prior to its first use, the apparatus shown in Fig 1 requires an initial biometric enrollment using for example the integrated fingerprint sensor 8 and recording an initial physiological reference value using physiological data sensor 9 such as the user's glucose or hemoglobin levels. To accomplish this, the apparatus is activated via an ON/OFF switch 18 (Fig 1). The user places his/her finger onto the finger guide 2 of the apparatus and after alignment with enclosure opening 4 the fingerprint sensor 8 records authentication templates followed by alignment with opening 5 the physiological sensor 9 can record the initial physiological template. This part of the method is referred to as "Enrollment". The processor 10 analyses the recorded information-using algorithm 11 and stores the biometric template in its data memory 12. The same method is followed after physiological data sensor 9 records physiological information and the processor 10 and algorithm 11 generate a physiological template for the user following spectral analysis of the reflected light waves from the user's subcutaneous tissue. The physiological template can also be stored in the memory 12 of the apparatus.

When the apparatus is to be used in continuous mode, a one-time authentication per day (or other pre-defined time range) is sufficient. This applies well if the user can wear the apparatus all day on his body. If the apparatus is used in a mode requiring periodic on and off-switching using ON/OFF transmission switch 18 or following an automatic shut off by the method of the apparatus caused by a security breach, a new identification is always required, referred to also as "Validation" since the apparatus will otherwise not work based on security considerations. In the course of a biometric validation the processor 10 generates a new template using algorithm 11 and compares it with already stored templates. In the course of physiological validation or enrollment the physiological data sensor 9 is activated and, following the spectral analysis, the new physiological template is compared to already stored templates or a new one being stored in memory 12 for later data transmission. Acceptance or rejection of the newly recorded biometric template can be shown on display 14, via LED at enclosure opening 4 or via integrated liquid crystal display or external graphic display (not shown). Alternatively an acoustic signal can indicate acceptance or rejection using an acoustic generator (not shown). The new physiological template or the result of a comparison between the stored and newly recorded physiological template can be displayed in similar format as described above.

After recording the biometric and/or physiological templates the processor 10 determines according to its configuration whether to store the physiological template or to forward it to the communication chip 15 for transmission to an external receiver (not shown). As part of the above data flow configuration the processor 10 determines whether the biometric and physiological templates are to be transmitted together or only the biometric template. The processor also decides whether the templates should be transmitted simultaneously or staggered and/or whether the biometric and physiological templates have to be inseparably transmitted. Transmission of the templates can be activated by the user of the apparatus as needed, for example by using transmission switch 18, automatically activated by the external receiver or executed in continuous mode. In case of special events such as a medical emergency, authorized individuals may activate transmission of some or all of the physiological data. Those individuals may be pre-defined or can use special codes to activate data transmission. Examples are doctors, EMS staff, security staff, military personnel etc. The processor 10 may respond to special external codes requesting selective data retrieval, updating the stored information or deleting selected or all information stored in the apparatus.

If the user of the apparatus enters the recording field of the external receiver, the receiver can be configured to automatically retrieve all or selected data from the entering user's apparatus by communicating such codes in secured ways to the communication chip 15 of the apparatus.

In a preferred embodiment of the method, physiological data is recorded continuously and compared in real time with stored templates or values. This is of great value to diabetics. The processor 10 can compare newly created templates with stored, pre-defined or externally imported value ranges to prevent unnecessary alarms or warnings.

The described embodiment of the apparatus is particularly valuable for preventive health care efforts and caretaking of medically infirm and elderly persons. The apparatus allows for a continuous and/or as needed supervision aided by the capability of the apparatus to inseparably link the biometric identification with the ongoing recording and evaluation of the same person's medical condition combined with an exceptionally high data transmission security. This special functionality set of apparatus carrying out the method of the invention replaces several individual apparatus otherwise needed to record, store and process the data recorded. The apparatus dramatically speeds the availability of those critical data, reduces cost and simplifies and eliminates many routine and costly jobs carried out be medical support staff. The present method can eliminate the constantly present danger of losing the link between the individual to the physiological data recorded for him or her. In addition, the method can be of critical help in the supervision of medical personnel and support staff during their performance of daily tasks: i.e. access to the operating room could be allowed only for medical staff whose stored data in the apparatus indicates compliance with all federal and local requirements such as completed training programs etc. Upon entry of the staff into the operating room, the receiver could request all data needed to determine whether this individual is authenticated to participate in the intended activity (surgery, baby care etc.) and is properly scheduled to attend.

The apparatus (and the corresponding method of the invention) can also be configured to access the users partial or entire medical record, for example when the user visits a medical facility, and store that record on the device. The user through the described biomedical authentication can then control access to the record on the device. Where the user visits several specialists, for example, the user could receive new information from an office, store that information and subsequently transmit the medical record from the device for the use of subsequent health care providers. Thus, the device can be configured to accommodate two-way communication between the users device and external devices. The device can also be configured to receive data from external physiological measuring devices, for example doctor's office blood pressure devices, heart monitors and the like. Configuring the device would include incorporating the communication protocols from the potential external measuring devices and providing for the selection of the protocols at the time of communication.

With respect to receiving data from external devices, nanotechnology based devices are being developed which can be sent into the human body to report on conditions of organs, deliver medication and even "report" where they are currently located. The term "sensing physiological characteristic data", as used herein, includes sensing signals from such nanoparticulate devices.

In a further embodiment (not shown) the apparatus for carrying out the method of the invention contains only one sensor module that is capable of recording biometric and physiological data, through an integration of sensor modules 8 and 9 within the apparatus. Such a sensor configuration is available from Lumidigm Inc., and can be integrated into the enclosure 1 of the apparatus. The Lumidigm sensor as outlined above is capable of recording biometric and physiological information of the user. While this sensor may not meet certain standards such as AFIS, it has being deployed already in many successful installations. The apparatus can employ only one sensor for biometric and physiological identification with the performance of such sensor being acceptable within a substantial set of application requirements. Such a sensor can result in further cost savings and reduction in size of the apparatus.

The apparatus can also be integrated with a GPS chip (not shown) adding the determination of the location of the living being in addition to his/her biometric identification and recording of physiological function and information.

Additional applications can be recognizable by a person of skill in the art based on the description and applying the invention. It is intended that the description and embodiment shown are merely examples and that the scope of the invention is determined by the following claims and their equivalent embodiments.

## Claims

1. A method for secure transmission of information regarding the identity of an individual and physiological characteristics of that individual the identity of an individual and physiological characteristics of that individual comprising the steps of:
sensing biometric characteristic data of the individual;
comparing said biometric characteristic data to characteristic data of predetermined registered individuals and determining whether said individual is registered;
sensing physiological characteristic data of said individual;
comparing said physiological characteristic data with a predetermined range of acceptable values for said physiological characteristic data; and
sensing a parameter characteristic of a security condition; comparing said parameter characteristic of a security condition with a predetermined range of acceptable values for said parameter transmitting a signal if said individual is registered, if said physiological characteristic data is within said predetermined range or not and if said parameter characteristic of a security condition is within said range of acceptable values for said parameter;
**characterized by**
determing that an external receiver has received said signal;
terminating transmitting if said signal has not been received for a predetermined amount of time and
resuming transmission on the condition that breach of said security condition has been rectified and that the individual reauthenticates with biometric information.

2. Method according to claim 1 wherein said physiological characteristic data are selected from the group consisting of heart rate data, blood pressure data and blood alcohol content data.

3. Method according to claim 1 or 2 wherein said security condition parameter is selected from the group consisting of temperature change, change in motion characteristics, lack of required training and change in skin to sensor distance, wherein a sensor forming part of an apparatus using the method in accordance with claim 1 or 2 senses a change of the capacitive field surrounding the skin of said individual upon a change in the skin to sensor distance.

4. Method according to claims 1 to 3 further including the step of encoding said signal before transmitting.

5. Method according to claims 1 to 4 including the step of sensing said physiological data over time.

6. Method according to claims 1 to 5 including the step of recording said physiological data over time.

7. Method according to claims 1 to 6 wherein said biometric data is selected from the group consisting of cutaneous patterns, facial feature patterns, voice patterns, human sweat and blood vessel patterns.

8. Method according to claims 1 to 7 wherein said transmitting of said signal is continuous.

9. Method according to claims 1 to 8 wherein said signal is transmitted through Near Field communication apparatus.

10. Method according to claims 1 to 9 wherein said signal is transmitted using the electrical conductivity of skin.

11. Method according to claims 1 to 10 wherein said signal includes said biometric characteristic data.

12. Method according to claims 1 to 11 wherein said signal includes said physiological data.

13. Method according to claims 1 to 12 wherein the first individual or the first number of individuals who are enrolling themselves biometrically are considered master users and wherein when external data transmission has been terminated under certain security conditions transmission can be resumed when one of the master users is first validated biometrically followed by the biometric validation of a current user.

## Patentansprüche

1. Verfahren zur sicheren Übertragung von die Identität eines Individuums und die physiologischen Eigenschaften dieses Individuums betreffenden Informationen, umfassend die Verfahrensschritte: Erfassen der biometrischen charakteristischen Daten des Individuums; Vergleich dieser biometrischen charakteristischen Daten mit charakteristischen Daten zuvor festgelegter, registrierter Individuen und Bestimmung, ob das Individuum registriert ist; Erfassen der physiologisch charakteristischen Daten des besagten Individuums; Vergleich dieser physiologisch charakteristischen Daten mit einem vorgegebenen Bereich akzeptabler Werte für derartige physiologisch charakteristische Daten; und Erfassen eines Parameters, welcher für ein Sicherheitserfordernis charakteristisch ist; Vergleich dieses für ein Sicherheitserfordernis charakteristischen Parameters mit einem für diesen Parameter vorgegebenen Bereich an akzeptablen Werten; Übertragung eines Signals, wenn das Individuum registriert ist, wenn die physiologisch charakteristischen Daten sich innerhalb des vorgegebenen Bereichs bewegen oder nicht und wenn sich besagter für ein Sicherheitserfordernis charakteristischer Parameter innerhalb des Bereichs an akzeptablen Werten für diesen Parameter befindet;
**gekennzeichnet durch** die Verfahrensschritte
Bestimmen, dass ein externer Empfänger das besagte Signal empfangen hat;
Beendigen der Signalübertragung, wenn das Signal für eine vorgegebene Zeitspanne nicht empfangen worden ist und
Wiederaufnahme der Signalübertragung unter der Bedingung, dass der Verstoß gegen das Sicherheitserfordernis berichtigt worden ist und dass sich das Individuum mit biometrischer Information erneut legitimiert.

2. Verfahren nach Anspruch 1, bei dem besagte physiologisch charakteristische Daten aus einer Gruppe von Daten ausgewählt werden, zu denen Daten betreffend den Herzschlag, den Blutdruck und den Alkoholgehalt im Blut gehören

3. Verfahren nach Anspruch 1 oder 2, bei dem der besagte, ein Sicherheitserfordernis betreffende Parameter aus einer Gruppe ausgewählt ist, zu denen eine Veränderung der Temperatur, eine Veränderung in der Bewegungscharakteristik, das Fehlen eines erforderlichen Trainings und ein Wechsel im Abstand zwischen der Haut und einem Sensor gehören, wobei ein Sensor, welcher Teil eines Geräts zur Durchführung des Verfahrens gemäß den Ansprüchen 1 oder 2 bildet, eine Veränderung im kapazitiven, die Haut des Individuums umgebenden Feld feststellt, wenn es zu einer Veränderung im Abstand zwischen der Haut und dem Sensor kommt.

4. Verfahren nach den Ansprüchen 1 bis 3 **gekennzeichnet durch** den weiteren Verfahrensschritt des Codierens des Signals vor der Übertragung.

5. Verfahren nach den Ansprüchen 1 bis 4 **gekennzeichnet durch** den Verfahrensschritt des Erfassens besagter physiologischer Daten über die Zeit.

6. Verfahren nach den Ansprüchen 1 bis 5 **gekennzeichnet durch** den Verfahrensschritt der Aufzeichnung der physiologischen Daten über die Zeit.

7. Verfahren nach den Ansprüchen 1 bis 6 , wobei besagte biometrische Daten aus der Gruppe ausgewählt werden, zu denen die Haut betreffende Muster, Muster im Gesichtsausdruck, Stimmmuster, den menschlichen Schweiß und die Blutgefäße betreffende Muster gehören.

8. Verfahren nach den Ansprüchen 1 bis 7, bei dem die Signalübertragung kontinuierlich erfolgt.

9. Verfahren nach den Ansprüchen 1 bis 8, bei dem die Signalübertragung mittels eines Nahfeld-Kommunikationsgerätes erfolgt.

10. Verfahren nach den Ansprüchen 1 bis 9, wobei das Signal unter Nutzung der elektrischen Leitfähigkeit der Haut übertragen wird.

11. Verfahren nach den Ansprüchen 1 bis 10, wobei das Signal besagte biometrische Daten enthält.

12. Verfahren nach den Ansprüchen 1 bis 11, wobei das Signal besagte physiologische Daten enthält.

13. Verfahren nach den Ansprüchen 1 bis 12, wobei das erste Individuum oder die erste Gruppe von Individuen, die sich biometrisch registrieren, als Hauptnutzer angesehen werden und wobei für den Fall, dass die externe Datenübertragung wegen gewisser Sicherheitserfordernisse beendet wurde, die Datenübertragung wieder aufgenommen werden kann, wenn sich einer der Hauptnutzer zunächst biometrisch legitimiert, gefolgt von der biometrischen Legitimierung eines aktuellen Nutzers.

## Revendications

1. Procédé de transmission sûre d'informations concernant l'identité d'un individu et les caractéristiques physiologiques de cet individu, comprenant les étapes suivantes :
détection de données caractéristiques biométriques de l'individu ;
comparaison de ces données caractéristiques biométriques avec les données caractéristiques d'individus enregistrés prédéterminés et vérification de la présence dudit individu dans les données enregistrées ;
détection de données caractéristiques physiologiques de l'individu ;
comparaison de ces données caractéristiques physiologiques avec une fourchette prédéterminée de valeurs acceptables pour les données caractéristiques physiologiques ; et
détection d'une caractéristique de paramètre d'une condition de sécurité comparaison de cette caractéristique de paramètre d'une condition de sécurité avec une fourchette prédéterminée de valeurs acceptables pour le paramètre ;
transmission d'un signal si l'individu est enregistré, si les données caractéristiques physiologiques se trouvent dans ladite fourchette prédéterminée ou non et si la caractéristique de paramètre d'une condition de sécurité se trouve dans la fourchette de valeurs acceptables pour le paramètre ;
**caractérisé en ce**
**qu'**il est déterminé qu'un destinataire extérieur a reçu le signal ;
**que** la transmission est interrompue si le signal n'a pas été reçu au cours d'une période de temps prédéterminée, et
**que** la transmission est rétablie à la condition que le non-respect de la condition de sécurité ait été rectifié et que l'individu soit une nouvelle fois authentifié à l'aide d'informations biométriques.

2. Procédé selon la revendication 1, sachant que les données caractéristiques physiologiques sont sélectionnées dans le groupe constitué de données sur la fréquence cardiaque, de données sur la tension artérielle et de données relatives au taux d'alcool dans le sang.

3. Procédé selon la revendication 1 ou 2, sachant que le paramètre de condition de sécurité est sélectionné dans un groupe constitué de données de changement de température, de changement de caractéristiques de déplacement, de manque d'entraînement nécessaire et de changement de distance entre la peau et le capteur, sachant qu'un capteur formant une partie d'un appareil utilisant le procédé selon la revendication 1 ou 2 détecte un changement du champ capacitif après un changement de distance entre la peau et le capteur.

4. Procédé selon les revendications 1 à 3 comprenant en outre l'étape de codage du signal avant sa transmission.

5. Procédé selon les revendications 1 à 4 comprenant en outre l'étape de détection des données physiologiques dans le temps.

6. Procédé selon les revendications 1 à 5 comprenant en outre l'étape d'enregistrement des données physiologiques dans le temps.

7. Procédé selon les revendications 1 à 6, sachant que les données biométriques sont sélectionnées dans un groupe constitué de modèles cutanés, de modèles de caractéristiques faciales, de modèles vocaux, de modèles de transpiration humaine et de vaisseaux sanguins.

8. Procédé selon les revendications 1 à 7, sachant que la transmission du signal est continue.

9. Procédé selon les revendications 1 à 8, sachant que le signal est transmis par un appareil de type Near Field Communication.

10. Procédé selon les revendications 1 à 9, sachant que le signal est transmis en utilisant la conductivité électrique de la peau.

11. Procédé selon les revendications 1 à 10, sachant que le signal comprend les données caractéristiques biométriques.

12. Procédé selon les revendications 1 à 11, sachant que le signal comprend les données physiologiques.

13. Procédé selon les revendications 1 à 12, sachant que le premier individu ou le premier nombre d'individus qui s'enregistrent avec leurs données biométriques sont considérés comme les utilisateurs de référence, et que, lorsque la transmission de données externes a été interrompue dans certaines conditions de sécurité, la transmission peut être rétablie lorsque les valeurs biométriques de l'un des utilisateurs de référence sont validées puis suivies par la validation biométrique d'un utilisateur actuel.
